# EUROPÄISCHE PATENTANMELDUNG

(11) **EP 0 848 254 A2**
(43) Veröffentlichungstag der Anmeldung: **17.06.1998**
(21) Anmeldenummer: 97119960.9
(22) Anmeldetag: 14.11.1997
(51) Int. Cl.: G01N 33/49, B01L 3/02, B01L 3/14, B01D 61/18, B01D 33/01

(54) **Verfahren zum automatischen Eindrücken eines Filters in ein Blutaufnahmegefäss**

(30) Priorität: 19.11.1996 DE 19647674
(71) Anmelder: Sarstedt AG & Co., 51582 Nümbrecht (DE)
(72) Erfinder: Sarstedt, Walter, 51582 Nümbrecht (DE)
(74) Vertreter: Pollmeier, Felix, Dipl.-Ing.

(57) **Zusammenfassung**

Mit einem Verfahren zum Eindrücken eines Filters (13) in ein Blutaufnahmegefäß (Probenröhrchen 1), bei dem Blut in dem Probenröhrchen (1) durch Zentrifugieren oder Absedimentieren in zwei Phasen, Serum bzw. Plasma (3) und Blutkuchen (2), getrennt und zwischen den Phasen eine Grenzschicht ausgebildet und/oder eine diese verstärkende Trennschicht (6) und/oder ein Trennkörper vorgesehen wird, ein in das Probenröhrchen (1) eingesetzter Filter (13) mit Aufnahmegefäß (14) automatisch durch ein Betätigungselement (11) in das Probenröhrchen bis zu einer bestimmten Position eingedrückt wird, wobei diese Position durch Ermittlung der Grenzschicht festgelegt wird, und/oder die Abwärtsbewegung des Betätigungselementes (11) durch Messung eines Druckanstiegs am Betätigungselement beim Auftreffen auf die Grenzschicht und/oder bei Erreichen eines definierten Füllvolumens des Aufnahmegefäßes (14) des Filters (13) durch Festlegung der Serum- bzw. Plasmaoberfläche und der zurückgelegten Wegstrecke gestoppt wird, und in jedem Fall zwischen der Oberfläche des Serum- bzw. Plasmafüllstandes in dem Aufnahmegefäß (14) und der Unterkante des Betätigungselementes (11) ein Luftpolster (15) aufrechterhalten wird, wird die Gefahr einer Verschleppung von Serum bzw. Plasma (3) und damit die Kontaminierung des Arbeitsbereiches vermieden sowie wegen des mehrfach zu verwendenden Betätigungselementes (11) der Materialeinsatz verringert.

## Beschreibung

Die Erfindung betrifft ein Verfahren zum Eindrücken eines Filters in ein Blutaufnahmegefäß, in der Regel ein zylindrisches Probenröhrchen, das an einem Ende geschlossen und am anderen Ende offen ist, wobei das einem Patienten entnommene Blut in dem Probenröhrchen durch Zentrifugieren oder Absedimentieren in zwei Phasen, nämlich Serum oder Plasma und den sich im unteren Teil des Röhrchens ansammelnden Blutkuchen getrennt wird.

Durch die DE 24 04 522 A ist eine Vorrichtung mit einem Filterkolben bekanntgeworden, der jeweils manuell in ein Probenröhrchen eingedrückt wird. Der Kolben weist ein als Gehäuse für den Filter dienendes becherförmiges Mittelteil auf, und zum Eindrücken des Filterkolbens wird von einer Bedienungsperson ein Betätigungselement in Form eines Aufnahmerohres verwendet, in das zuvor der Kolben mit dem Filter eingesetzt worden ist. Beim Einschieben des Filterkolbens strömt das Serum durch den Filter bzw. den Kolben hindurch in den Raum oberhalb des Kolbens mit dem Filter. Der Filter verhindert bei einem ohne Gelschicht abgefüllten Probenröhrchen, daß Fibrinfäden oder -nester im Serum vorhanden sind.

Die Bedienungsperson muß bei der Entnahme darauf achten, daß einerseits die Grenzschicht zwischen Serum/Pasma und Blutkuchen nicht überschritten, d.h. das Eindrücken des Filterkolbens möglichst bei Erreichen der Grenzschicht beendet wird, andererseits aber für die sich anschließenden Untersuchungen eine ausreichende Menge an gefiltertem Serum bzw. Plasma aufgenommen wird. Hierbei hat sich als nachteilig herausgestellt, daß bei einem hohen Probendurchsatz die Konzentration der Bedienungsperson nachläßt, was zu fehlerhaftem Probenmaterial führen kann; zudem muß das Betätigungselement bzw. Aufnahmerohr nach jeder Anwendung verworfen bzw. entsorgt werden, um eine Verschleppung der an dem in die Flüssigkeit eintauchenden Kolben unvermeidlich anhaftenden kontaminierten Flüssigkeit (Serum oder Plasma) von einer Probe zur nächsten Probe zu vermeiden. Außerdem besteht die Gefahr, daß beim Herausnehmen des Betätigungselementes von diesem Tropfen unkontrolliert abfallen und im Arbeitsbereich verspritzen, so daß es zu einer Kontaminierung der Umgebung kommen kann. Schließlich steht weiterhin das Betätigungselement undefiniert und mit solch großer Länge aus dem Probenröhrchen vor, daß es sich ergreifen und aus dem Probenröhrchen herausziehen läßt.

Der Erfindung liegt die Aufgabe zugrunde, ein Verfahren zum Eindrücken eines Filters in ein Blutaufnahmegefäß bzw. Probenröhrchen zu schaffen, mit dem sich die vorgenannten Nachteile vermeiden lassen und das auch den Materialeinsatz verringert.

Diese Aufgabe wird erfindungsgemäß durch die in dem Patentanspruch angegebenen Maßnahmen gelöst, die es erlauben, einen Filter automatisch in ein Probenröhrchen einzudrücken und die Abwärtsbewegung an einem bestimmten Punkt automatisch zu stoppen. Hierzu erhält eine mit einem Eindrückstempel bzw. Betätigungselement ausgerüstete automatische Filtereindrückvorrichtung ein Erkennungssystem, z.B. eine Ultraschall- oder Durchlichteinrichtung, mit der sich die Grenzschicht erkennen läßt, die durch Vorsehen einer Trennschicht in Form von Gel oder eines Trennkörpers noch deutlicher zu unterscheiden ist. Alternativ oder ergänzend zur Grenzschichtermittlung und des damit wegabhängig geregelten Eindrückvorgangs läßt sich die Abwärtsbewegung des Betätigungselementes und damit der Eindrückvorgang druckabhängig regeln. Sobald sich nämlich am Betätigungelement ein Druckanstieg messen läßt, der über den von dem Serum bzw. Plama erzeugten Widerstand hinausgeht, was beim Auftreffen des Filters auf das Gel, den Trennkörper oder den Blutkuchen der Fall ist, übersteigt der Druck einen vorher festgelegten Wert, woraufhin der Eindrückvorgang beendet wird.

Da die von den Eigenschaften des Blutes (Hämatokrit) abhängige Höhe und Lage der einzelnen abgetrennten Phasen von Probe zu Probe verschieden ist, wird erfindungsgemäß weiterhin vorgeschlagen, den Spiegel bzw. die Oberfläche des Serums bzw. Plasmas und damit den Füllstand festzustellen, so daß bei ungünstiger Konstellation, d.h. bei einem gegenüber dem Volumen des Aufnahmegefäßes des Filters höheren Serum- bzw. Plasmaanteil der Eindrückweg einen von den Abmessungen der Probenröhre und insbesondere dem Volumen des Aufnahmegefäßes abhängiger Weg nicht überschreitet.

Sollte der Flüssigkeitsanteil von Serum oder Plasma somit größer als das Aufnahmevolumen des kragenlosen, in das Probenröhrchen eintauchenden Aufnahmegefäßes sein, wird die Abwärtsbewegung gestoppt und damit eine Berührung zwischen dem Serum oder Plasma und dem Betätigungselement vermieden; hierbei kann es somit vorkommen, daß der Filter in seiner Endposition eine Lage mit einem Abstand oberhalb zur Grenzschicht einnimmt.

In jedem Fall wird erfindungsgemäß sichergestellt, daß ein Überlaufen von Serum oder Plasma aus dem Aufnahmegefäß verhindert wird und oben in dem Aufnahmegefäß stets ein Luftpolster bleibt, d.h. das Aufnahmegefäß nicht bis zu seinem Rand gefüllt wird. Da ein Überlaufen des Serums aus dem Aufnahmegefäß auch bei maximaler Wegstrecke bzw. Eindrücktiefe des Filters verhindert wird, kann das Betätigungselement bzw. der Einschiebstempel der automatischen Eindrückvorrichtung nicht in Kontakt mit dem Serum bzw. Plasma gelangen, so daß keine Gefahr einer Kontamination, wie insbesondere durch Verschleppung besteht, und das Betätigungselement im Grunde ohne ausgewechselt werden zu müssen beliebig oft verwendet werden kann. Es braucht daher auch nur ein kontaminiertes Teil entsorgt zu werden, nämlich das Probenröhrchen mit dem darin einschließlich Aufnahmegefäß verbleibenden Filter. Aufgrund des erfindungsgemäß erreichten, von Kontaminationen freien Betriebes beim automatischen Eindrücken des Filters und der wiederholten Einsetzbarkeit des Betätigungselementes eignet sich das erfindungsgemäße Verfahren nicht nur für eine als Einzelgerät eingesetzte, separate Eindrückvorrichtung, sondern insbesondere auch für den Einsatz in einem aus der DE 40 32 048 C2 bekannten automatischen Probenverarbeitungs- und -verteilungsgerät. Die Integration einer nach dem erfindungsgemäßen Verfahren betriebenen automatischen Eindrückvorrichtung in einem solchen Probenverteilerablauf eignet sich auch deshalb besonders gut, weil der Filter mit dem kragenlosen Aufnahmegefäß zumeist völlig in das Primärröhrchen eintaucht, keinesfalls aber undefiniert übersteht, so daß ein ungehindertes Weitertakten der Probenröhrchen gewährleistet ist.

Weitere Einzelheiten und Vorteile der Erfindung ergeben sich aus der nachfolgenden Beschreibung, in der schematisch einige Ausführungsbeispiele des Gegenstandes der Erfindung dargestellt sind. Es zeigen:
- Fig. 1: als Einzelheit ein Probenröhrchen mit den darin in Blutkuchen und Serum bzw. Plasma abgetrennten Phasen;
- Fig. 2: ein Probenröhrchen gemäß Fig. 1 mit einem darin zur Verstärkung der Grenzschicht zwischen dem Blutkuchen und dem Serum bzw. Plasma eingesetzten Trennkörper;
- Fig. 3: ein Probenröhrchen gemäß Fig. 1 mit sich zwischen dem Blutkuchen und dem Serum bzw. Plasma befindender Gelschicht und Anordnung des Röhrchens in einer schematisch gezeigten automatischen Eindrückvorrichtung zum Einschieben eines Filters mit Aufnahmegefäß in das Probenröhrchen;
- Fig. 4: den bei dem Probenröhrchen gemäß Fig. 3 bis auf die Gelschicht eingedrückten Filter; und
- Fig. 5: den bei übermäßigem Serum- bzw. Plasmaanteil wegabhängig und vom Füllvolumen des Aufnahmegefäßes des Filters bestimmt in das Röhrchen eingedrückten Filter.

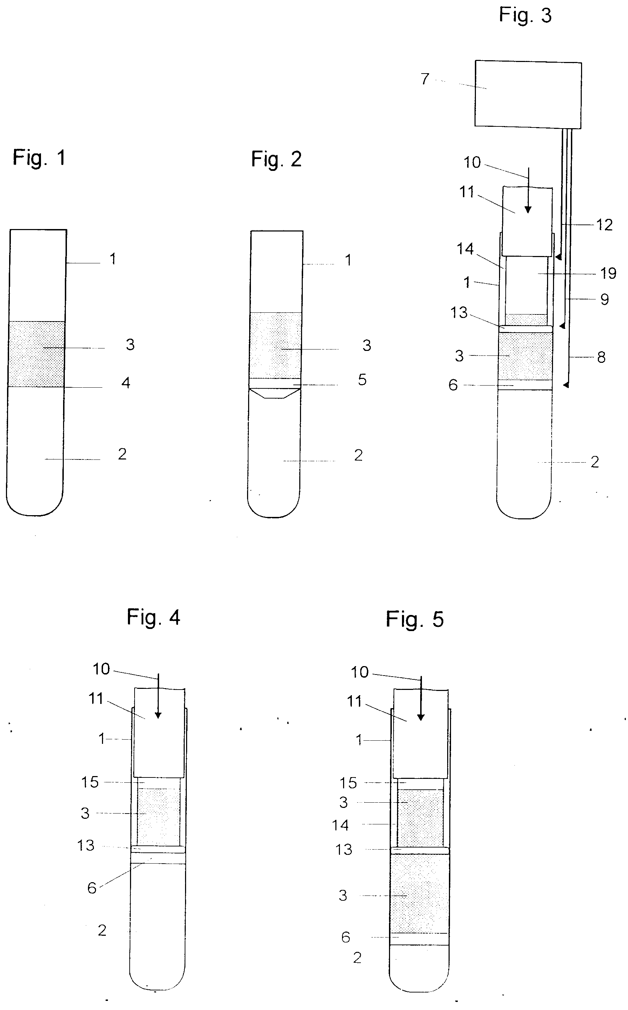

Wie in Fig. 1 gezeigt, ist eine in einem Probenröhrchen 1 vorhandene Blutmenge durch Zentrifugieren oder Absedimentieren in zwei Phasen getrennt worden, nämlich den sich unten in dem Röhrchen ansammelnden Blutkuchen 2 und das sich darüber befindende Serum bzw. Plasma 3, wobei im Übergangsbereich der beiden Phasen die Grenzschicht 4 liegt. Diese läßt sich gemäß Fig. 2 durch einen zwischengeschalteten Trennkörper 5 bzw. eine sich dazwischen aufbauende Gelschicht 6 (vgl. die Fig. 3 bis 5) verstärken und damit zur besseren Erkennung deutlicher hervorheben.

Zur Entnahme der für sich anschließende Untersuchungen benötigten Menge Serum bzw. Plasma 3 wird das Probenröhrchen 1 in eine in Fig. 3 im wesentlichen lediglich als Black-Box dargestellte automatische Eindrückvorrichtung 7 eingesetzt; diese ist mit einem optischen Grenzschicht-Erkennungsmittel 8, einer Meßeinrichtung 9 zur Feststellung der Oberfläche und damit des Füllstandes von entweder Serum oder Plasma 3 in dem Probenröhrchen 1 sowie einem in Pfeilrichtung 10 (vgl. Fig. 3) absenkbaren Betätigungselement 11 ausgerüstet, dem eine Druckmeßeinrichtung 12 zugeordnet ist. Zur Entnahme von Serum bzw. Plasma 3 wird mittels des Betätigungselementes 11 ein zuvor in das Probenröhrchen 1 eingesetzter Filter 13 eingedrückt; der Filter 13 ist mit einem gleichzeitig als Niederdrückstempel dienenden, kragenlosen Aufnahmegefäß 14 verbunden, in das das Serum bzw. Plasma 3 mit dem zunehmenden Eindrücken durch das Filter 13 hindurch entsprechend allmählich ansteigt, wie in Fig. 3 zu erkennen.

Abhängig von der mit dem Erkennungsmittel 8 festgestellten Grenzschicht werden Filter und Aufnahmegefäß 13 bzw. 14 nur so weit eingedrückt, daß eine ausreichende Menge - sofern vorhanden - aufgenommen wird, ohne daß es allerdings zu einem Überlaufen kommt; es wird stets ein Luftpolster 15 (vgl. die Fig. 4 und 5) aufrechterhalten. Das Betätigungselement 11 der automatischen Eindrückvorrichtung 7 kommt daher niemals in Kontakt mit dem Serum bzw. Plasma 3, denn bei Erreichen eines definierten Füllvolumens des Aufnahmegefäßes 14, was sich aus der zurückgelegten Wegstrecke ergibt, wird der Eindrückvorgang beendet und das Betätigungselement 11 aus dem Probenröhrchen 1 herausgefahren. Wenn - wie in Fig. 5 gezeigt - der aus dem Blut gewonnene Serum- bzw. Plasmaanteil sehr viel größer als das Füllvolumen des Aufnahmegefäßes 14 ist, nimmt der Filterkolben 13 einen gegebenenfalls großen Abstand von der Gelschicht 6 (und/oder einem Trennkörper 5, vgl. Fig. 2) ein.

Gemäß Fig. 4 wird die Beendigung des Eindrückvorganges aufgrund eines mit der Druckmeßeinrichtung 12 gemessenen Druckanstiegs ausgelöst. Denn - ein entsprechend geringer Anteil an gewonnenem Serum bzw. Plasma 3 vorausgesetzt, da der Eindrückvorgang ansonsten wie beschrieben wegabhängig bereits beendet worden wäre - sobald der Filter 13 auf die im Ausführungsbeispiel nach Fig. 4 vorhandene Gelschicht 6 (und/oder einen Trennkörper 5) trifft:, ergibt sich ein gegenüber dem von dem Plasma bzw. Serum 3 ausgeübter höherer Widerstand, und ab einem bestimmten daraus resultierenden Druck wird der Eindrückvorgang beendet und das Betätigungselement 11 aus dem Probenröhrchen 1 herausgefahren. Die für die Untersuchungen benötigte Menge an Plasma oder Serum 3 wird danach mit einem Saugrohr oder eine Probennehmernadel aus dem Aufnahmegefäß 14 entnommen, wobei diese Entnahmemittel nicht in den Blutkuchen bzw. die Gelschicht eintauchen können.

## Patentansprüche

1. Verfahren zum Eindrücken eines Filters in ein Blutaufnahmegefäß (Probenröhrchen),
- bei dem Blut in dem Probenröhrchen durch Zentrifugieren oder Absedimentieren in zwei Phasen, Serum bzw. Plasma und Blutkuchen, getrennt und
- zwischen den Phasen eine Grenzschicht ausgebildet und/oder eine diese verstärkende Trennschicht (Gel) und/oder ein Trennkörper vorgesehen wird,
- ein in das Probenröhrchen eingesetzter Filter mit Aufnahmegefäß automatisch durch ein Betätigungselement in das Probenröhrchen bis zu einer bestimmten Position eingedrückt wird, wobei
- diese Position durch Ermittlung der Grenzschicht festgelegt wird,
- und/oder die Abwärtsbewegung des Betätigungselementes durch Messung eines Druckanstiegs am Betätigungselement beim Auftreffen auf die Grenzschicht und/oder bei Erreichen eines definierten Füllvolumens des Aufnahmegefäßes des Filters durch Feststellung der Serum- bzw. Plasmaoberfläche und der zurückgelegten Wegstrecke gestoppt wird,
- und in jedem Fall zwischen der Oberfläche des Serum- bzw. Plasmafüllstandes in dem Aufnahemegefäß und der Unterkante des Betätigungselementes ein Luftpolster aufrechterhalten wird.
